# FASCICULE DE BREVET EUROPEEN

(11) **EP 0 619 114 B1**
(45) Date de publication et mention de la délivrance du brevet: **21.08.1996**
(21) Numéro de dépôt: 94400485.2
(22) Date de dépôt: 08.03.1994
(51) Int. Cl.: A61K 7/135

(54) **Composition décolorante sous forme granulee utilisable pour la décoloration des cheveux et procédé de préparation de ladite composition**
Bleichzusammensetzung in Form eines Granulats zum Bleichen von Haaren und Verfahren zur Herstellung
Bleaching composition in granulate form for hair bleaching and process for preparing it

(30) Priorité: 05.04.1993 FR 9303995
(43) Date de publication de la demande: 12.10.1994
(73) Titulaire: L'OREAL, F-75008 Paris (FR)
(72) Inventeur: Millequant, Jean-Marie, F-94100 Saint-maur (FR); Tricaud, Caroline, F-95240 Cormeilles en Parisis (FR); Gaboriaud, Anne, F-93340 Le Raincy (FR)
(74) Mandataire: Peuscet, Jacques

(56) Documents cités:
- WO-A-92/03120
- DE-A- 2 023 922
- DE-A- 3 434 468

## Description

La présente invention concerne une composition décolorante sous forme granulée à base de dérivés peroxydés, utilisable notamment pour la décoloration des cheveux, et un procédé de préparation de ladite composition.

Il est connu de décolorer les cheveux à l'aide d'une pâte obtenue en mélangeant, au moment de l'application sur les cheveux, une composition décolorante à base de dérivés peroxydés avec de l'eau ou, mieux encore, avec de l'eau oxygénée. La composition décolorante est, de façon connue, constituée d'un dérivé peroxydé, généralement un persulfate ou un perborate de sodium, de potassium ou d'ammonium et parfois un sel d'acide percarboxylique ou un peroxyde, par exemple de baryum ou de strontium. Ces compositions contiennent également, de façon connue, des agents fortement alcalins tels que des métasilicates, des phosphates ou des carbonates alcalins ou alcalino-terreux ; elles peuvent éventuellement contenir d'autres additifs : des agents de contrôle du dégagement d'oxygène lors du mélange avec l'eau oxygénée, tels que le carbonate de magnésium ou la magnésie , des agents tensioactifs, tels que les sulfates d'alcools gras, les alkylsulfates et les alkylbenzène sulfonates ; des agents épaississants, tels que les dérivés de cellulose, par exemple la carboxyméthylcellulose, l'amidon et ses dérivés, les gommes de guar, de xanthane et les alginates ; des colorants bleus ou violets et des parfums. De telles compositions décolorantes sont décrites, par exemple, dans "The science of hair care" par C. Zviak, Marcel Dekker Inc. 1986, pages 225 et 226.

La composition décolorante est souvent utilisée sous forme de poudre de faible granulométrie, ce qui permet une dissolution facile et rapide dans l'eau oxygénée. Mais ces compositions pulvérulentes présentent plusieurs inconvénients. En premier lieu, les compositions pulvérulentes sont constituées de poudres ayant des densités apparentes différentes et, au cours de leur manipulation et de leur stockage, il se forme une ségrégation des constituants, les plus lourds se rassemblant à la partie inférieure de l'emballage dans lequel la composition est contenue et les plus légers à la partie supérieure ; par conséquent, lors du prélèvement de la composition pour la mélanger avec l'eau oxygénée, les volumes prélevés à la partie supérieure de l'emballage et ceux prélevés à la partie inférieure ont des compositions différentes et donc un pouvoir de décoloration différent. En second lieu, les compositions sous forme pulvérulente émettent, lors de leur manipulation, des poussières qui contiennent des dérivés peroxydés et sont, par conséquent, fortement irritantes pour les poumons.

Pour résoudre ce problème, on a déjà proposé de mettre la composition décolorante sous forme granulée.

Selon WO-A 92/03120, on a proposé de granuler un persulfate et de le mélanger avec des particules, éventuellement granulées, des différents autres constituants de la composition. La granulation du persulfate peut se faire soit par pulvérisation et séchage d'une solution aqueuse de persulfate contenant éventuellement des agents tensioactifs ou des agents épaississants solubles dans l'eau, soit par pulvérisation d'une solution d'agent tensioactif ou d'agent épaississant sur un lit mobile de persulfate solide. Ce procédé, s'il peut permettre, a priori, d'éviter la formation de poussières irritantes de persulfate ne permet certainement pas de résoudre le problème de la ségrégation des particules des différents constituants de la composition décolorante.

Dans FR-A 2 044 324, on a proposé de granuler l'ensemble des constituants de la composition décolorante pulvérulente à l'aide d'un liant : la polyvinylpyrrolidone ou le glucose en solution dans un milieu aqueux, hydroalcoolique ou alcoolique. On résoud ainsi le problème de la ségrégation des différents constituants de la composition décolorante puisque les différents constituants sont présents dans un même granulé. Mais on a constaté que l'emploi de polyvinylpyrrolidone seule, comme liant, donne des granules durs mais cependant friables qui, par frottement répété, forment de fines poussières qui sont susceptibles de voler dans l'atmosphère. De plus, on obtient des granules ayant une granulométrie élevée comprise entre 1 et 6 mm, ce qui augmente le temps de dissolution et donne, par mélange avec l'eau oxygénée, une pâte qui reste longtemps granuleuse et dont l'application est désagréable ; de plus, cette pâte risque de donner par application sur les cheveux une décoloration non homogène et non reproductible.

Selon la présente invention, on a trouvé qu'en n'utilisant plus un seul liant mais deux liants, le premier étant un homopolymère et/ou un copolymère de vinylpyrrolidone ou l'alcool polyvinylique et le second un polyalkylèneglycol, non seulement on évitait le problème de ségrégation des différents constituants, mais encore on obtenait des granules qui ne forment pratiquement pas de poussière par frottement et, qui, lorsque leur granulométrie est comprise entre 65 µm et 800 µm, se dissolvent facilement dans l'eau oxygénée pour donner une pâte homogène et onctueuse facile à appliquer sur une chevelure. En outre, il faut noter qu'il est possible de broyer les granules ayant une granulométrie supérieure à 800 µm sans qu'il se forme de poussière irritante.

La présente invention a, par conséquent, pour objet une composition décolorante granulée, utilisable pour la décoloration des cheveux, dans laquelle les granules ont été obtenus par granulation d'un mélange de ses différents constituants pulvérulents au moyen d'un agent de granulation, caractérisée par le fait que ledit agent de granulation comprend deux liants: un premier liant choisi dans le groupe formé par l'alcool polyvinylique, les homo- et les copolymères de vinylpyrrolidone et leurs mélanges et un second liant choisi dans le groupe formé par les polyalkylèneglycols, dont le groupe alkylène est en C₂ à C₄ et qui ont un poids moléculaire compris entre 200 et 30 000 et leurs mélanges, les granules ayant une granulométrie comprise entre 65 µm et 800 µm.

Selon la présente invention, le premier liant peut donc être non seulement constitué par les homopolymères de vinylpyrrolidone utilisés dans FR-A 2 044 324, mais également par les copolymères de vinylpyrrolidone et par l'alcool polyvinylique. Le premier liant est, de préférence, l'alcool polyvinylique.

Le second liant peut être tout polyalkylèneglycol (ou mélange de polyalkylèneglycols) dont le groupe alkylène est en C₂-C₄, ayant un poids moléculaire compris entre 200 et 30 000. On utilise, avantageusement, un polypropylèneglycol ayant un poids moléculaire compris entre 2 000 et 8 000 ou un polyéthylèneglycol ayant un poids moléculaire compris entre 200 et 4 000, plus particulièrement compris entre 200 et 600.

La teneur globale en premier et second liants ne dépasse pas, de préférence 25 % en poids par rapport au poids total de la composition. La concentration de chacun des liants est avantageusement comprise entre 2 et 20 % en poids par rapport au poids total de la composition : cette concentration est, de préférence, de 2 à 8 % en poids pour le premier liant, notamment lorsque celui-ci est un alcool polyvinylique ou un copolymère vinylpyrrolidone/acétate de vinyle, et de 4 à 15 % en poids pour le second liant, notamment lorsque celui-ci est le polypropylèneglycol.

Selon l'invention, la composition granulée doit avoir une granulométrie comprise entre 65 µm et 800 µm. En effet, lorsque la granulométrie est supérieure à 800 µm, les granules commencent à se dissoudre difficilement dans l'eau oxygénée et leur dissolution peut ne pas être totale ; la pâte obtenue est granuleuse et n'est pas homogène. Lorsque la composition a une granulométrie inférieure à 65 µm, il se forme des poussières irritantes lors de la manipulation de la composition décolorante sous forme granulée.

Selon la présente invention, on a également trouvé que se dissolvent plus facilement dans l'eau oxygénée, les compositions décolorantes granulées contenant au moins un agent lubrifiant de la famille des dérivés de cellulose, de préférence la carboxyméthylcellulose de sodium ou l'hydroxypropylméthylcellulose et/ou au moins un agent lubrifiant de la famille des stéarates alcalins ou alcalino-terreux ou de polyol. Les stéarates de polyol sont, de préférence, choisis parmi le stéarate de glycol et le stéarate de glycérol. On utilise, de préférence, un mélange de stéarate de potassium et de stéarate de glycérol. En effet, en présence d'agent(s) lubrifiant(s), les granules sont moins durs et moins friables. La quantité de dérivé(s) de cellulose présente comme lubrifiant est, avantageusement, comprise entre 1 et 10 % en poids par rapport au poids total de la composition, de préférence, 2 à 6 % ; la quantité de stéarate(s) présente est, avantageusement, comprise entre 0,1 et 5 % en poids par rapport au poids total de la composition, de préférence, 0,2 et 1 % ; la quantité globale d'agent lubrifiant est avantageusement comprise entre 1 et 10 % en poids par rapport au poids total de la composition.

La granulation du mélange pulvérulent contenant les différents constituants de la composition décolorante peut se faire en mettant en solution dans un solvant à la fois le premier liant constitué par un homo- ou un copolymère de vinylpyrrolidone ou l'alcool polyvinylique (ou leurs mélanges) et le second liant constitué par un polyalkylèneglycol (ou un mélange de polyalkylèneglycols) et en pulvérisant la solution obtenue sur un lit mobile du mélange pulvérulent, avantageusement homogénéisé au préalable, le solvant étant ensuite éliminé.

Mais on préfère un autre mode de granulation dans lequel on introduit le premier liant dans le mélange pulvérulent, on homogénéise de préférence le mélange, on pulvérise le second liant, en solution dans un solvant, sur un lit mobile du mélange obtenu et on élimine le solvant. Ce procédé permet d'obtenir des granules de granulométrie plus homogène et, en particulier, évite la formation de fines ayant une granulométrie inférieure à 65 µm. La quantité de granules ayant une granulométrie supérieure à 800 µm est faible et ils peuvent être rebroyés sans que, pratiquement, il se produise des fines. Pour régler la granulométrie à la valeur désirée, on peut agir sur la durée de granulation.

Le solvant peut être tout solvant compatible avec le(s) liant(s) qui y est (sont) dissous. On évite cependant d'utiliser un milieu aqueux (de l'eau ou un milieu hydroalcoolique, par exemple) car la présence d'eau peut amorcer la décomposition des dérivés peroxydés et, lorsque la composition décolorante contient un dérivé d'ammonium (par exemple du persulfate d'ammonium), provoquer un dégagement d'ammoniac. Le solvant est, de préférence, un alcanol en C₁-C₄ ou les chlorures de méthylène ou d'éthylène, ou un mélange de ces composés.

Lorsqu'un agent lubrifiant est utilisé, celui-ci est introduit dans le mélange pulvérulent.

Le lit mobile de mélange pulvérulent sur lequel est pulvérisée la solution de liant peut être tout lit mobile connu pour la granulation : il peut être un lit obtenu dans une cuve à l'aide d'agitateurs notamment de pales ou de batteurs, un lit obtenu dans un tambour rotatif, ou un lit obtenu à l'aide d'un courant gazeux ascendant, tel qu'un lit fluidisé.

Le mélange pulvérulent est, de préférence, homogénéisé avant la pulvérisation de la solution de liant. Cette homogénéisation peut être effectuée dans un mélangeur annexe ou dans le lit mobile avant pulvérisation. Par exemple, dans le cas où on effectue la granulation dans une cuve, on introduit dans la cuve tous les constituants pulvérulents du mélange, on homogénéise le mélange par agitation dans la cuve avant de pulvériser la solution de liant, et on poursuit l'agitation, après en avoir éventuellement modifié les conditions, pour maintenir le mélange à granuler sous forme de lit mobile pendant la pulvérisation et la granulation. Lorsqu'on granule en lit fluidisé, le mélange pulvérulent à granuler peut être homogénéisé par agitation dans une cuve annexe, avant introduction dudit mélange dans le granulateur à lit fluidisé.

La granulation peut être effectuée en discontinu ou en continu.

Après granulation, on sépare éventuellement, d'une part, les fines ayant des dimensions inférieures à 65 µm, ces fines pouvant être recyclées, et d'autre part, les particules ayant des dimensions supérieures à 800 µm, qui sont broyées sans provoquer la formation de poussières irritantes ; le produit obtenu après broyage et classification est introduit selon sa granulométrie, soit dans le mélange pulvérulent, soit dans le produit fini.

Les exemples donnés ci-après, à titre illustratif et nullement limitatif, permettront de mieux comprendre l'invention.

### I - Préparation des granules

### EXEMPLES 1 à 7

On utilise un granulateur "ROTO 50 P" commercialisé par la société italienne "ZANCHETTA & C" comportant une cuve de 50 litres, muni d'un couvercle équipé de couteaux rotatifs et d'une buse de pulvérisation, muni d'un agitateur tripale en fond de cuve, d'une double enveloppe, d'une pompe à vide et d'un système de basculement de la cuve à ± 90°.

On introduit dans la cuve 15 kg du mélange pulvérulent contenant le premier liant (alcool polyvinylique (PVA) vendu sous la dénomination de "POWAL 224" par la société "KURARAYS" ou copolymère de vinylpyrrolidone et d'acétate de vinyle (PVP/VA) vendu sous la dénomination "LUVISKOL VA 64 POUDRE" par la société "BASF") et ayant la composition donnée (en % en poids) dans les treize premières lignes du tableau I. On homogénéise le mélange pendant 5 minutes à l'aide du tripale tournant à 200 tours/minute et des couteaux tournant à 1 000 tours/minute. On arrête les couteaux et on poursuit l'agitation à l'aide du tripale après avoir amené sa vitesse de rotation à 160 tours/minute.

Pendant ce temps, on prépare une solution du deuxième liant (polyéthylèneglycol de poids moléculaire 400 (PEG 400) et/ou polypropylèneglycol de poids moléculaire 2 000 (PPG 2 000)) dans le dichlorométhane ; cette solution est obtenue en mélangeant le second liant et le dichlorométhane à raison de 1,5 kg de second liant pour un litre de dichlorométhane.

Dès que l'on a arrêté les couteaux comme indiqué ci-dessus, on pulvérise la solution de second liant sur le lit pulvérulent contenu dans le granulateur en quantité suffisante pour introduire dans le granulateur le poids de second liant désiré tel qu'indiqué en % en poids aux quatorzième et quinzième lignes du tableau I.

Lorsque la pulvérisation est terminée, on amène la vitesse de rotation des pales à 200 tours/minute et on fait tourner les couteaux à 1 200 tours/minute jusqu'à obtention d'un pic de puissance des moteurs de l'agitation, ce qui correspond à la fin de la granulation.

On met en route la pompe à vide, on bascule de 90° le granulateur (l'axe de la cuve est alors presque horizontal) et on chauffe la double enveloppe de façon à obtenir une température ne dépassant pas 40°C dans le lit de granules de façon à éliminer le solvant.

### EXEMPLE 8

On utilise le même granulateur "ROTO 50 P" que dans les exemples 1 à 7.

On introduit dans la cuve 15 kg du mélange pulvérulent ayant la composition donnée (en % en poids) dans les treize premières lignes du tableau I. On homogénéise le mélange pendant 5 minutes à l'aide du tripale tournant à 200 tours/mn et des couteaux tournant à 1 000 tours/mn. On arrête les couteaux et on poursuit l'agitation à l'aide du tripale après avoir amené sa vitesse de rotation à 160 tours/mn. Pendant ce temps, on prépare une solution de deux liants (polyéthylèneglycol de poids moléculaire 400 (PEG 400) et du copolymère de vinylpyrrolidone et d'acétate de vinyle vendu sous la dénomination "LUVISKOL VA 64 POUDRE" par la société "BASF") dans le dichlorométhane.

Dès que l'on a arrêté les couteaux, on pulvérise la solution des deux liants sur le lit pulvérulent contenu dans le granulateur en quantité suffisante pour introduire dans le granulateur le poids de liants désiré tel qu'indiqué en % aux quatorzième et quinzième lignes du tableau I.

On termine l'opération comme indiqué dans les exemples 1 à7.

### II - Essais sur les granules obtenus selon les exemples 1 à 8

On a effectué les tests suivants :
a) Mesure de la granulométrie dans l'appareil de calibration commercialisé sous la dénomination "FC" par la société italienne "ZANCHETTA & C"
b) Test de dissolution des grains.
   On prélève 10 g de poudre décolorante granulée que l'on met dans un bol non métallique. On empâte à l'aide d'une spatule avec 10 g d'eau oxygénée à 30 volumes. On détermine le temps de dissolution totale par observation à l'oeil nu du cataplasme obtenu, le temps maximum étant de 30 minutes.
c) Mesure de la volatilité
   On pèse dans des pots en verre 10 g de poudre granulée des différents échantillons de poudre granulée.
   On agite de la même façon (trois allers et retours de bas en haut et vice versa) et on évalue visuellement le nuage de poussière qui subsiste en prenant en compte la densité du nuage, sa persistance et la taille des particules en suspension. On définit une échelle de volatilité qui va de 0 à 5, la valeur 0 correspondant à une poudre non volatile et 5 à une poudre très volatile.

Les résultats de ces différents essais sont donnés dans les trois dernières lignes du tableau I ci-après.

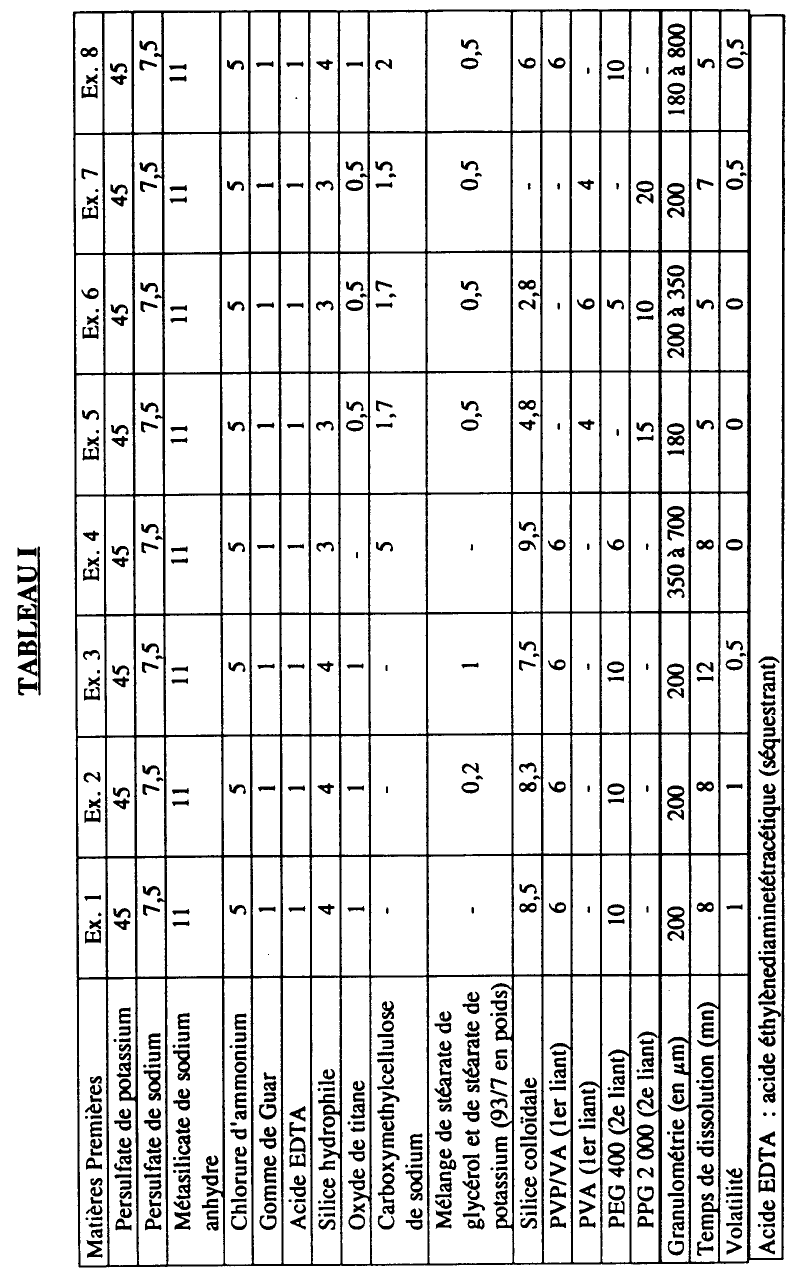

### EXEMPLES 9 et 10

On a effectué des essais comparatifs avec des poudres granulées par le procédé décrit pour les exemples 1 à 7, en n'utilisant qu'un seul liant ; un polymère polyvinylpyrrolidone (PVP) vendu sous la dénomination "LUVISKOL K 30" par la société "BASF" ou un polyéthylèneglycol de poids moléculaire 400 (PEG 400). On a effectué sur les granules obtenus les mêmes essais que pour les exemples 1 à 8. La formulation de la composition granulée (en % en poids) et les résultats des essais sont donnés dans le tableau II ci-après :

**TABLEAU II**

| Matières premières | Exemple 9 | Exemple 10 |
|---|---|---|
| Persulfate de potassium | 40 | 50 |
| Persulfate de sodium | 9 | 7,5 |
| Métasilicate de sodium anhydre | 10 | 10,8 |
| Chlorure d'ammonium | 5 | 4 |
| Gomme de Guar | - | 1 |
| Acide éthylènediaminetétracétique | 2 | 1 |
| Silice hydrophile | - | 3 |
| Oxyde de titane | - | - |
| Carboxyméthylcellulose de sodium | 5 | - |
| Mélange de stéarate de glycérol et de stéarate de potassium (93/7 en poids) | - | 0,5 |
| Silice colloïdale | 17 | 10,2 |
| PVP | 12 | - |
| PEG 400 | - | 12 |
| Granulométrie (en µm) | > 1 000 | 65 à 1 000 |
| Temps de dissolution (mn) | > 60 | 30 |
| Volatilité | 1 | 1,5 |

Ces essais montrent que lorsqu'on utilise un seul liant, les temps de dissolution des granules sont plus élevés, la granulomérie des granules est trop élevée ou trop dispersée et la formation de poussière est relativement élevée.

## Revendications

1. Composition décolorante granulée, utilisable notamment pour la décoloration des cheveux, dans laquelle les granules ont été obtenus par granulation d'un mélange de ses différents constituants pulvérulents au moyen d'un agent de granulation, caractérisée par le fait que l'agent de granulation comprend deux liants : un premier liant choisi dans le groupe formé par les homo- et les copolymères de vinylpyrrolidone, l'alcool polyvinylique et leurs mélanges et un second liant choisi dans le groupe formé par les polyalkylèneglycols , dont le groupe alkylène est en C₂-C₄ et qui ont un poids moléculaire compris entre 200 et 30 000, et leurs mélanges, les granules ayant une granulométrie comprise entre 65 µm et 800 µm.

2. Composition selon la revendication 1, caractérisée par le fait que le premier liant est un alcool polyvinylique.

3. Composition selon l'une des revendications 1 ou 2, caractérisée par le fait que le second liant est un polypropylèneglycol ayant un poids moléculaire compris entre 200 et 8 000.

4. Composition selon l'une des revendications 1 ou 2, caractérisée par le fait que le second liant est un polyéthylèneglycol ayant un poids moléculaire compris entre 200 et 4 000 et, de préférence, entre 200 et 600.

5. Composition selon les revendications 1 et 4, caractérisée par le fait que la concentration de chacun des liants est comprise entre 2 et 20 %.

6. Composition selon l'une des revendications 1 à 5, caractérisée par le fait que le premier liant est présent à une concentration comprise entre 2 et 8 %.

7. Composition selon l'une des revendications 1 à 6, caractérisée par le fait qu'elle contient au moins un agent lubrifiant pris dans le groupe formé par les dérivés de cellulose et les stéarates.

8. Composition selon la revendication 7, caractérisée par le fait que l'agent lubrifiant comporte au moins un dérivé de cellulose choisi dans le groupe formé par la carboxyméthylcellulose de sodium et l'hydroxypropylméthylcellulose.

9. Composition selon l'une des revendications 7 ou 8, caractérisée par le fait que la quantité de dérivé(s) de cellulose présent(s) comme agent lubrifiant représente de 1 à 10 % en poids du poids total de la composition.

10. Composition selon la revendication 7, caractérisée par le fait que l'agent lubrifiant est choisi dans le groupe formé par les stéarates alcalins, les stéarates alcalino-terreux et les stéarates de polyol.

11. Composition selon l'une des revendications 7 ou 10, caractérisée par le fait que la quantité de stéarate(s) présent(s) comme agent lubrifiant est comprise entre 0,1 et 5 % en poids par rapport au poids total de la composition.

12. Procédé de préparation de la composition selon l'une des revendications 1 à 11, caractérisé par le fait qu'on ajoute le premier liant aux différents constituants pulvérulents du mélange à granuler, on homogénéise le mélange, on pulvérise le second liant, en solution dans un solvant, sur un lit mobile du mélange précédemment obtenu et on élimine le solvant.

13. Procédé selon la revendication 12, caractérisé par le fait qu'on choisit le solvant du second liant dans le groupe formé par les alcanols en C₁-C₄, le chlorure de méthylène, le chlorure d'éthylène et leurs mélanges.

14. Procédé selon l'une des revendications 12 ou 13, destiné à préparer une composition selon la revendication 7, caractérisé par le fait qu'on ajoute l'(ou les) agent(s) lubrifiant(s) aux constituants pulvérulents du mélange à granuler.

15. Procédé selon l'une des revendications 12 à 14, caractérisé par le fait qu'on obtient le lit mobile du mélange à granuler dans une cuve à l'aide d'agitateur(s) ou dans un tambour rotatif.

16. Procédé selon l'une des revendication 12 à 14, caractérisé par le fait qu'on obtient le lit mobile du mélange à granuler par fluidisation dans un courant gazeux ascendant.

17. Procédé selon l'une des revendications 12 à 16, caractérisé par le fait qu'on élimine le solvant thermiquement et/ou sous pression réduite.

## Claims

1. Granulated bleaching composition which may especially be used for bleaching hair, in which the granules have been obtained by granulation of a mixture of its various pulverulent constituents by means of a granulation agent, characterized in that the granulation agent comprises two binders: a first binder chosen from the group formed by polyvinyl alcohol, homo- and copolymers of vinylpyrrolidone and their mixtures and a second binder chosen from the group formed by polyalkylene glycols in which the alkylene group is C₂ to C₄ and which have a molecular weight between 200 and 30,000 and their mixtures, the granules having a particle size between 65 µm and 800 µm.

2. Composition according to Claim 1, characterized in that the first binder is a polyvinyl alcohol.

3. Composition according to either of Claims 1 and 2, characterized in that the second binder is a polypropylene glycol having a molecular weight between 200 and 8000.

4. Composition according to either of Claims 1 and 2, characterized in that the second binder is a polyethylene glycol having a molecular weight between 200 and 4000 and preferably between 200 and 600.

5. Composition according to Claims 1 to 4, characterized in that the concentration of each of the binders is between 2 and 20%.

6. Composition according to one of Claims 1 to 5, characterized in that the first binder is present in a concentration between 2 and 8%.

7. Composition according to one of Claims 1 to 6, characterized in that it contains at least one lubricating agent taken from the group formed by cellulose derivatives and stearates.

8. Composition according to Claim 7, characterized in that the lubricating agent contains at least one cellulose derivative chosen from the group formed by sodium carboxymethyl cellulose and hydroxypropylmethyl cellulose.

9. Composition according to either of Claims 7 and 8, characterized in that the quantity of cellulose derivative(s) present as lubricating agent represents from 1 to 10% by weight of the total weight of the composition.

10. Composition according to Claim 7, characterized in that the lubricating agent is chosen from the group formed by alkali metal stearates, alkaline-earth metal stearates and polyol stearates.

11. Composition according to either of Claims 7 and 10, characterized in that the quantity of stearate(s) present as lubricating agent is between 0.1 and 5% by weight relative to the total weight of the composition.

12. Process for the preparation of the composition according to one of Claims 1 to 11, characterized in that the first binder is added to the various pulverulent constituents of the mixture to be granulated, the mixture is homogenized, the second binder dissolved in a solvent is sprayed onto a moving bed of the previously obtained mixture and the solvent is removed.

13. Process according to Claim 12, characterized in that the solvent for the second binder is chosen from the group formed by C₁-C₄ alkanols, methylene chloride, ethylene chloride and their mixtures.

14. Process according to either of Claims 12 and 13, intended for the preparation of a composition according to Claim 7, characterized in that the lubricating agent(s) is (are) added to the pulverulent constituents of the mixture to be granulated.

15. Process according to one of Claims 12 to 14, characterized in that the moving bed for the mixture to be granulated is obtained in a tank with the aid of (a) stirrer(s) or in a rotating drum.

16. Process according to one of Claims 12 to 14, characterized in that the moving bed for the mixture to be granulated is obtained by fluidization in a stream of ascending gas.

17. Process according to one of Claims 12 to 16, characterized in that the solvent is removed thermally and/or under reduced pressure.

## Patentansprüche

1. Bleichzusammensetzung in Form von Granulat, die insbesondere zum Bleichen von Haaren verwendbar ist und in der das Granulat durch Granulierung einer Mischung ihrer verschiedenen pulverförmigen Bestandteile mit Hilfe eines Granulierungsmittels erhalten wurde, dadurch gekennzeichnet, daß das Granulierungsmittel zwei Bindemittel umfaßt: ein erstes Bindemittel, das aus der Gruppe ausgewählt ist, die von den Vinylpyrrolidon-Homo- und Kopolymeren, Polyvinylalkohol und ihren Mischungen gebildet ist, und ein zweites Bindemittel, das aus der Gruppe ausgewählt ist, die von den Polyalkylenglykolen mit einer C₂-C₄-Alkylengruppe und einem Molekulargewicht von 200 bis 30 OOO und ihren Mischungen gebildet ist, wobei das Granulat eine Korngröße von 65 bis 800 µm hat.

2. Zusammensetzung nach Anspruch 1, dadurch gekennzeichnet, daß das erste Bindemittel ein Polyvinylalkohol ist.

3. Zusammensetzung nach einem der Ansprüche 1 oder 2, dadurch gekennzeichnet, daß das zweite Bindemittel ein Polypropylenglykol mit einem Molekulargewicht von 200 bis 8 000 ist.

4. Zusammensetzung nach einem der Ansprüche 1 oder 2, dadurch gekennzeichnet, daß das zweite Bindemittel ein Polyethylenglykol mit einem Molekulargewicht von 200 bis 4 000 und vorzugsweise 200 bis 600 ist.

5. Zusammensetzung nach den Ansprüchen 1 und 4, dadurch gekennzeichnet, daß die Konzentration jedes der Bindemittel 2 bis 20 % beträgt.

6. Zusammensetzung nach einem der Ansprüche 1 bis 5, dadurch gekennzeichnet, daß das erste Bindemittel in einer Konzentration von 2 bis 8 % vorliegt.

7. Zusammensetzung nach einem der Ansprüche 1 bis 6, dadurch gekennzeichnet, daß sie mindestens ein Gleitmittel enthält, das aus der von den Zellulosederivaten und den Stearaten gebildeten Gruppe ausgewählt ist.

8. Zusammensetzung nach Anspruch 7, dadurch gekennzeichnet, daß das Gleitmittel mindestens ein Zellulosederivat umfaßt, das aus der von Natriumkarboxymethylzellulose und Hydroxypropylmethylzellulose gebildeten Gruppe ausgewählt ist.

9. Zusammensetzung nach einem der Ansprüche 7 oder 8, dadurch gekennzeichnet, daß die Menge des bzw. der als Gleitmittel vorhandenen Zellulosederivate 1 bis 10 Gew.-% des Gesamtgewichts der Zusammensetzung darstellt.

10. Zusammensetzung nach Anspruch 7, dadurch gekennzeichnet, daß das Gleitmittel aus der Gruppe ausgewählt ist, die von den alkalischen Stearaten, den erdalkalischen Stearaten und den Polyolstearaten gebildet ist.

11. Zusammensetzung nach einem der Ansprüche 7 oder 10, dadurch gekennzeichnet, daß die Menge des bzw. der als Gleitmittel vorhandenen Stearate 0,1 bis 5 Gew.-%, bezogen auf das Gesamtgewicht der Zusammensetzung, beträgt.

12. Verfahren zur Herstellung der Zusammensetzung nach einem der Ansprüche 1 bis 11, dadurch gekennzeichnet, daß man das erste Bindemittel den verschiedenen pulverförmigen Bestandteilen der zu granulierenden Mischung beigibt, man die Mischung homogenisiert, man das zweite Bindemittel in Lösung in einem Lösungsmittel auf ein bewegliches Bett der zuvor erhaltenen Mischung sprüht und man das Lösungsmittel entfernt.

13. Verfahren nach Anspruch 12, dadurch gekennzeichnet, daß man das Lösungsmittel des zweiten Bindemittels aus der Gruppe auswählt, die von den C₁-C₄-Alkanolen, Methylenchlorid, Ethylenchlorid und ihren Mischungen gebildet ist.

14. Verfahren nach einem der Ansprüche 12 oder 13, das zur Herstellung einer Zusammensetzung nach Anspruch 7 bestimmt ist, dadurch gekennzeichnet, daß man das oder die Gleitmittel den pulverförmigen Bestandteilen der zu granulierenden Mischung beigibt.

15. Verfahren nach einem der Ansprüche 12 bis 14, dadurch gekennzeichnet, daß man das bewegliche Bett der zu granulierenden Mischung in einem Behälter mit Hilfe eines oder mehrerer Rührer oder in einer rotierenden Trommel erhält.

16. Verfahren nach einem der Ansprüche 12 bis 14, dadurch gekennzeichnet, daß man das bewegliche Bette der zu granulierenden Mischung durch Fluidisierung in einem aufsteigenden Gastrom erhält.

17. Verfahren nach einem der Ansprüche 12 bis 16, dadurch gekennzeichnet, daß man das Lösungsmittel thermisch und/oder unter vermindertem Druck entfernt.
